Europäisches Patentamt

European Patent Office

Office européen des brevets

(19)

(11) **EP 0 771 872 A1**

(12) # DEMANDE DE BREVET EUROPEEN

(43) Date de publication:
**07.05.1997 Bulletin 1997/19**

(51) Int Cl.6: **C12N 15/10**, C12N 15/66,
C12N 15/70

(21) Numéro de dépôt: **96401959.0**

(22) Date de dépôt: **12.09.1996**

(84) Etats contractants désignés:
**DE FR GB IT**

(30) Priorité: **15.09.1995 FR 9510872**

(71) Demandeur: **CENTRE NATIONAL DE LA RECHERCHE SCIENTIFIQUE (CNRS)**
**75794 Paris Cédex 16 (FR)**

(72) Inventeurs:
- **Masson, Jean-Michel**
  **31400 Toulouse (FR)**
- **Boe, Florence**
  **Bât. A. Renoir, 31500 Toulouse (FR)**

(74) Mandataire: **Vialle-Presles, Marie José et al**
**Cabinet Orès,**
**6, Avenue de Messine**
**75008 Paris (FR)**

(54) **Procédé de polymèrisation de séquences d'acides nucléiques et ses applications**

(57) Ce procédé comprend essentiellement les étapes suivantes : (1) coupure d'un vecteur de clonage circulaire comprenant deux sites de restriction, a et b, donnant des extrémités cohésives compatibles entre elles et deux sites de restriction c et d, proches des sites de restriction a et b, à l'aide d'au moins une enzyme de restriction, apte à effectuer ladite coupure au niveau des sites a et b, (2) introduction par ligation de la séquence nucléique à polymériser dans le vecteur linéarisé obtenu en (1), entre les deux sites de restriction, a et b (vecteur 1x), (3) coupure du vecteur recircularisé obtenu en (2), à l'aide d'au moins une enzyme de restriction, apte à effectuer ladite coupure au niveau des sites de restriction c et d. (4) introduction par ligation, dans le vecteur linéarisé obtenu en (3), entre les deux sites de restriction c et d, d'un gène codant pour un ARNt suppresseur (vecteur 1x+s), (5) coupure du vecteur obtenu en (4) à l'aide d'au moins une enzyme de restriction, apte à effectuer ladite coupure au niveau des sites a et d, (6) introduction par ligation du fragment a-d obtenu en (5) (fragment 1x+s) entre les sites b et d d'un vecteur lx tel qu'obtenu en (2), après coupure de ce vecteur 1x au niveau desdits sites b et d (obtention du vecteur 2x+s), (7) introduction par ligation du fragment obtenu en (6) (fragment 2x+s) entre les sites b et d d'un vecteur 1x tel qu'obtenu en (2), après coupure de ce vecteur 1x telle que définie en (6)(obtention du vecteur 3x+s), (8) répétition de l'étape (7) par insertion du fragment nx+s obtenu en dernier lieu dans un vecteur lx, jusqu'à obtention d'une séquence comportant n+1 fragments identiques à la séquence nucléique à polymériser, (9) transformation d'une souche bactérienne avec le vecteur obtenu en (8) et sélection des souches présentant le caractère mut et (10) extraction du polymère (n+1)x, à partir desdites souches sélectionnées.

EP 0 771 872 A1

**Description**

La présente invention est relative à un procédé de polymérisation de séquences d'acides nucléiques ainsi qu'à ses applications, notamment en tant que marqueur de taille d'acide nucléique (ADN, par exemple), pour la construction de gènes codant pour des protéines hautement répétitives et pour l'inclusion de séquences répétitives d'acide nucléique et plus particulièrement d'ADN dans des vecteurs ou des séquences génomiques.

Il existe un certain nombre de méthodes pour la production d'acide nucléique en quantité importante, à partir de petites quantités d'échantillons d'acide nucléique ; on peut citer en particulier :

- les méthodes de clonage d'une séquence d'acide nucléique dans un système convenable, dans lesquelles une séquence d'acide nucléique est insérée dans un vecteur, qui est utilisé pour transformer une cellule hôte appropriée. Lorsque la cellule hôte est mise en culture, le vecteur est répliqué et en conséquence, de nombreuses copies de la séquence d'acide nucléique recherchée sont produites (Sambrook J. et al., *Molecular cloning : a laboratory manual,* 2ème éd., 1989, Cold Spring Harbor Laboratory).
- les méthodes d'amplification *in vitro,* telles que la PCR (réaction de polymérisation en chaîne) (SAIKI et al., Science, 1985, 230, 1350-1354).

Toutefois ces méthodes ne sont pas adaptées à l'obtention de polymères répétitifs d'une séquence ADN double-brin, de n'importe quelle taille (de quelques dizaines de nucléotides à quelques milliers de nucléotides). En outre, l'obtention de polymères répétitifs d'une séquence d'acide nucléique par polymérisation *in vitro,* suivie de la séparation des séquences en fonction de leur taille, avec ou sans coupures de restriction ou par des méthodes dérivées de la PCR, s'avère particulièrement délicate et difficile à mettre en oeuvre de manière industrielle.

En conséquence, la présente Demande s'est donné pour but de pourvoir à un procédé d'amplification de séquences d'acide nucléique, indépendant de la taille ou de la nature de la séquence, alors que les méthodes rappelées ci-dessus, entraînent des limitations liées soit à la nature de la séquence, soit à la taille du fragment à amplifier.

La présente invention a pour objet un procédé d'amplification ou de polymérisation d'une séquence nucléique, notamment d'une séquence d'ADN double-brin par une méthode de clonage, lequel procédé est caractérisé en ce qu'il comprend essentiellement les étapes suivantes :

(1) coupure d'un vecteur de clonage circulaire comprenant deux sites de restriction, $\underline{a}$ et $\underline{b}$, donnant des extrémités compatibles entre elles et deux sites de restriction $\underline{c}$ et $\underline{d}$, proches des sites de restriction $\underline{a}$ et $\underline{b}$, à l'aide d'au moins une enzyme de restriction, apte à effectuer ladite coupure au niveau des sites $\underline{a}$ et $\underline{b}$ ; au sens de l'invention, on entend par proche, le fait que les sites $\underline{c}$ et $\underline{d}$ doivent être du même côté de $\underline{a}$ ou $\underline{b}$, à au moins 6 paires de bases, de préférence à quelques dizaines de paires de bases de distance de $\underline{a}$ ou $\underline{b}$ ; en fait, il n'y a pas de distance maximale imposée par la méthode,

(2) introduction par ligation de la séquence nucléique à polymériser dans le vecteur linéarisé obtenu en (1), entre les deux sites de restriction, $\underline{a}$ et $\underline{b}$ (obtention du vecteur 1x, c'est-à-dire contenant une seule copie de la séquence à polymériser),

(3) coupure du vecteur recircularisé obtenu en (2), à l'aide d'au moins une enzyme de restriction, apte à effectuer ladite coupure au niveau des sites de restriction $\underline{c}$ et $\underline{d}$

(4) introduction par ligation, dans le vecteur linéarisé obtenu en (3), entre les deux sites de restriction $\underline{c}$ et $\underline{d}$, d'un gène codant pour un ARNt suppresseur (obtention du vecteur 1x+s, contenant une seule copie de la séquence à polymériser et le gène codant pour un ARNt suppresseur) et sélection des clones portant l'insert 1x+s dans la bonne orientation, par transformation de bactéries convenables,

(5) coupure du vecteur obtenu en (4) à l'aide d'au moins une enzyme de restriction, apte à effectuer ladite coupure au niveau des sites $\underline{a}$ et $\underline{d}$,

(6) introduction par ligation du fragment $\underline{a}$-$\underline{d}$ obtenu en (5) (fragment 1x+s) entre les sites $\underline{b}$ et $\underline{d}$ d'un vecteur 1x tel qu'obtenu en (2), après coupure de ce vecteur 1x à l'aide d'au moins une enzyme de restriction, apte à effectuer ladite coupure au niveau desdits sites $\underline{b}$ et $\underline{d}$ (obtention du vecteur 2x+s) et sélection des clones portant l'insert 2x+s dans la bonne orientation, par transformation de bactéries convenables,

(7) introduction par ligation du fragment obtenu en (6) (fragment 2x+s) entre les sites $\underline{b}$ et $\underline{d}$ d'un vecteur 1x tel qu'obtenu en (2), après coupure de ce vecteur 1x telle que définie en (6)(obtention du vecteur 3x+s) : l'obtention du polymère 3x se fait donc de la même manière qu'à l'étape (6), en introduisant le fragment 2x+s dans le vecteur 1x,

(8) répétition de l'étape (7) par insertion du fragment nx+s obtenu en dernier lieu dans un vecteur 1x, jusqu'à obtention d'une séquence comportant n+1 fragments identiques à la séquence nucléique à polymériser,

(9) transformation d'une souche bactérienne avec le vecteur obtenu en (8), laquelle souche bactérienne comporte une mutation (mut⁻) au moins dans un gène essentiel, de préférence à la fois dans un gène essentiel et dans un gène rapporteur, de telle sorte que son développement ne soit possible qu'en présence dudit ARNt suppresseur (c'est-à-dire en présence du vecteur nx+s) et sélection des souches présentant le caractère mut+ et

(10) extraction du polymère (n+1)x, à partir desdites souches sélectionnées.

Un cycle de clonage comporte l'insertion d'une séquence nx+s (avec n ≥ 2) dans un vecteur 1x ; à chacun de ces cycles, la jonction entre les fragments nx+1 et 1x, via les extrémités compatibles des sites a et b est telle que ne subsistent, effectivement, que les sites a et b aux extrémités du nouveau fragment polymérisé (n+1)x.

Le procédé selon la présente invention présente un certain nombre d'avantages :

- il permet d'amplifier ou de polymériser n'importe quelle séquence d'acide nucléique, (procédé indépendant de la nature de la séquence de l'ADN à polymériser) ; ceci est particulièrement avantageux pour réaliser des marqueurs de poids moléculaire, dont la migration est parfaitement conforme aux lois physico-chimiques (pas de défaut de migration, lié à la séquence), alors qu'avec les méthodes de l'Art antérieur exposées ci-dessus, il y a souvent un déséquilibre dans la représentation des bandes de différentes tailles ;
- il se prête particulièrement bien à la polymérisation de fragments de petite taille, souvent difficiles à cloner par les méthodes conventionnelles ; ceci est particulièrement important pour ce qui concerne les marqueurs de taille pour la PCR.

A titre d'exemple, le procédé selon l'invention permet en particulier de polymériser des séquences d'origine naturelle ou synthétique, telles que les séquences codant pour des protéines hautement répétitives, les séquences de régulation ou d'interaction avec des protéines se fixant à l'ADN ou l'ARN, d'origine virale ou cellulaire (sites de fixation des activateurs ou des répresseurs de l'expression des gènes, origines de réplication procaryotes ou eucaryotes).

Conformément à l'invention :

- les sites de restriction sont de préférence sélectionnés,

    . pour ce qui concerne les sites de restriction a et b, de préférence différents et aux extrémités compatibles, parmi tous les sites correspondant à des enzymes coupant l'ADN à bouts francs (Pvu II, Eco RV, Hinc II, Sma I, Sna BI, Ssp I, etc...), les sites de coupure des enzymes donnant des extrémités cohésives compatibles, mais dont la fusion ne régénère pas un site qui peut être recoupé par l'une des deux enzymes (donc pas d'enzymes avec un site de reconnaissance à quatre bases), tels que Eco RI et Mun I ; Apa LI et Sfc I ; Bam HI et Bcl I, Bgl II ou Bst YI ; Sal I et Xho I ; Nco I et Bsp HI ou Afl III ; Xma I et Age I ; Ngo MI et Bsp EI ; Xba I et Nhe I, Avr II ou Spe I ; Bss HII et Mlu I ou Dsa I ; Bst BI et Cla I, Acc I, Nar I, ou Psp 1406 I ; Pst I et Nsi I ;
    . pour ce qui concerne les sites de restriction c et d, de préférence différents des sites a et b, parmi n'importe quel site de restriction, à condition qu'il ne soit pas inclus dans la séquence de l'ARNt suppresseur ; en variante, le site b et le site c sont confondus ;

- ledit ARNt suppresseur est sélectionné parmi les ARNt suppresseurs d'ocre, d'opale, frameshift ou d'ambre, de préférence parmi un ARNt suppresseur d'ambre (JM Masson et al., Gene, 1986, 179-183 ; LG Kleina et al., J. Mol. Biol., 1990, **213,** 705-717 ; J. Normanly et al., J. Mol. Biol., 1990, **213**, 719-726 ; J. Normanly et al., Proc. Natl. Acad. Sci. USA, 1986, **83**, 6548-6552) ;
- ladite souche bactérienne est de préférence une souche *d'Escherichia coli* portant une mutation ambre à la fois dans un gène essentiel (argE, par exemple) et dans un gène rapporteur (lacZ, par exemple).

Le procédé conforme à l'invention inclut également le cas où le fragment de départ est déjà un fragment polymérisé, le terme 1x signifiant dans ce cas : « une unité à polymériser ».

En variante la souche bactérienne et notamment la souche d'*E. coli* est, en outre, *recA,* c'est-à-dire déficiente pour la recombinaison homologue ; en effet la construction de ce genre de polymère dans *E. coli* peut donner lieu à des phénomènes de recombinaison et de délétions, en particulier lorsque n > 10.

Conformément à l'invention, le couple suppresseur-souche sélectionnée fait que lorsque, à l'issue d'un clonage, une colonie pousse sur milieu sans arginine et est bleue, on a la certitude d'avoir à faire au bon recombinant, donc au bon insert, puisque c'est l'insert qui est porteur du marqueur de sélection.

Un tel procédé trouve notamment application :

1) Dans la préparation de marqueurs de taille d'ADN : la polymérisation d'une séquence d'ADN comprise entre deux sites de restriction telle qu'elle est décrite ci-dessus, permet de construire des échelles régulières de molécules d'ADN. Ces échelles sont utilisées comme « marqueurs » de taille lors de la migration de fragments d'ADN issus de séquençage ou d'une analyse par restriction.

Pour obtenir une telle échelle, il suffit de combiner les n vecteurs portant chacun un nombre défini de copies de la séquence initiale (de 1 à n) et de les couper tous par les deux enzymes compatibles a et b, qui encadrent la séquence polymérisée.

En variante, on peut procéder à la coupure des différents vecteurs, séparément, puis procéder au mélange des produits issus des différentes coupures.

L'avantage de cette méthode est que l'on peut doser les différents éléments, de façon à ce que certaines bandes soient sur-représentées par rapport à l'ensemble (par exemple, les bandes de 500, 1000 et 1500 pb, dans une échelle de 100 en 100), ce qui fournira un repère interne de taille.

Un autre avantage de la méthode est que l'on peut ainsi construire les échelles de taille que l'on souhaite, à la base près. On peut aussi faire des échelles ne comportant pas tous les éléments d'une série (100, 200, 400, 500, 700, 900, 1000 etc...), ce qui est éventuellement plus lisible.

2) Dans la construction de gènes codant pour des protéines hautement répétitives. Un certain nombre de protéines hautement répétitives ont des applications biotechnologiques potentielles importantes. C'est le cas des collagènes, des soies, de la kératine, de l'élastine et de nombreuses protéines d'adhésion ou de protéines non naturelles répétitives.

3) L'inclusion de séquences répétitives d'ADN dans des vecteurs ou des génomes : de telles séquences peuvent servir d'étiquettes pour isoler, caractériser ou purifier des fragments d'ADN par interaction avec d'autres séquences nucléotidiques ou avec des protéines se fixant spécifiquement à ces séquences. De telles séquences sont également particulièrement adaptées à la synthèse et à l'utilisation des séquences régulant la transcription des gènes dans les systèmes d'expression eucaryotes.

Outre les dispositions qui précèdent, l'invention comprend encore d'autres dispositions, qui ressortiront de la description qui va suivre, qui se réfère à des exemples de mise en oeuvre du procédé objet de la présente invention ainsi qu'aux dessins annexés, dans lesquels :

- les figures 1 et 2 illustrent le procédé de polymérisation de séquences d'acides nucléiques selon l'invention ; figure 1 : procédé général ; figure 2 : application dudit procédé à une séquence RRE dans un plasmide BSKS,
- la figure 3 représente le plasmide BSKS,
- la figure 4 représente le résultat de la polymérisation du RRE, après coupure par les enzymes Sal I et Xho I et électrophorèse en gel d'acrylamide-SDS (gradient d'acrylamide 8-25 %), puis coloration au nitrate d'argent,
- la figure 5 représente l'hybridation des oligonucléotides RRE1xa et b pour former l'élément minimal de liaison du RRE, et
- la figure 6 représente les clones 55 et 96 sur un film autoradiographique.

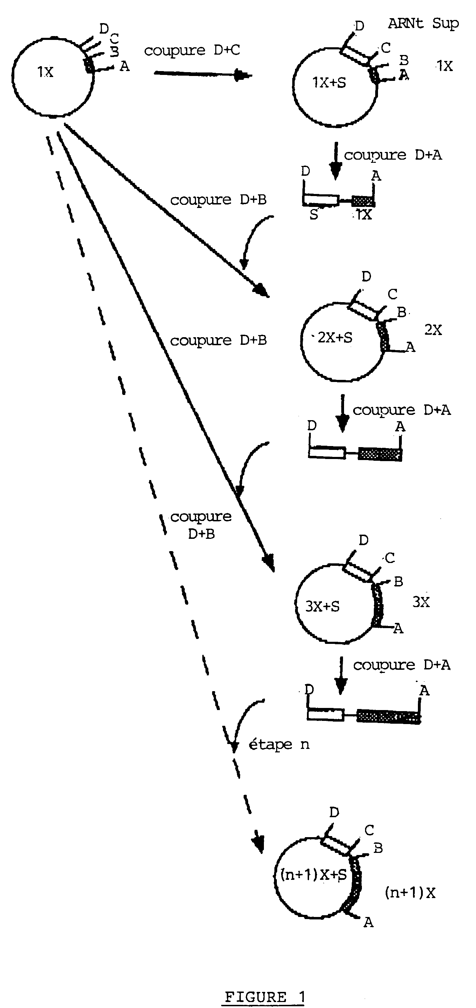

De manière générale, le procédé selon l'invention est illustré à la figure 1 : la séquence à polymériser est d'abord clonée entre deux sites de restriction aux extrémités compatibles dans un vecteur de clonage (sites A et B) produisant le vecteur 1X. Ensuite, une cassette comprenant la séquence ARNt ambre suppresseur avec son promoteur et le terminateur de transcription est cloné de manière adjacente (entre les sites C et D) à la séquence 1X pour obtenir le vecteur 1X+S qui est utilisé pour transformer une souche mutée ambre telle que par exemple XAC-1.

Le vecteur 2X+S est obtenu par coupure du fragment 1X+S avec les enzymes de restriction A et D et son clonage entre les sites B et D du vecteur 1X. La croissance des transformants sur un milieu minimum permet de sélectionner uniquement les constructions correctes.

De la même manière, le vecteur 3X+S est obtenu par clonage du fragment A/D 2X+S, dans un vecteur 1X entre les sites B et D et sélection des transformants pour la présence du gène suppresseur.

Le vecteur 3X+S contient trois copies adjacentes de la séquence à polymériser, dans la même orientation, entre deux sites de restriction unique A et B dans la mesure où les sites présents entre les copies sont détruits par la procédure de clonage et que le clonage asymétrique permet que seule la séquence dans la bonne orientation soit insérée. Chaque fois que l'étape de clonage est répétée, une unité de la séquence est ajoutée à la séquence polymérisée ($\rightarrow$ n+1).

Il doit être bien entendu, toutefois, que ces exemples sont donnés uniquement à titre d'illustration de l'objet de l'invention, dont ils ne constituent en aucune manière une limitation.

**EXEMPLE 1 : Mise en oeuvre du procédé pour préparer une séquence polymérique, à base de la séquence d'ARN cible de Rev, le RRE : obtention de la séquence polymérique RRE6x dans une souche d'E.coli XAC-1.**

A. Matériel

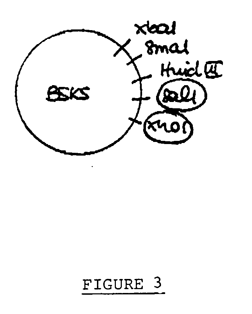

\* Séquence RRE :
Le RRE est une séquence de 234 nucléotides, présente dans le gène *env* du virus HIV-1, capable de former un ARN de structure codant pour la protéine exprimée par le RRE (M.H. MALIM et al., Nature, 1989, **338**, 254-257).
\* Plasmide BSKS (voir figure 3) :

Il est commercialisé par la société Stratagène sous l'appellation pBluescript KS+.
* Souche d'E. coli XAC-1 :
Celle-ci est notamment décrite dans la publication au nom de JM. MASSON et al. (Gene, 1986, **47**, 179-183).
* gène de l'ARNt$^{Phé}$ suppresseur d'*E. coli* :
La construction de ce gène est décrite dans J. Normanly et al. (Proc. Natl. Acad. Sci. USA, précité).

B. Méthodes

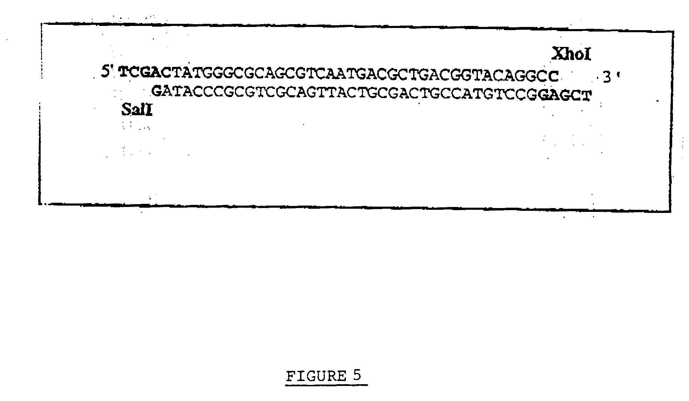

- Construction de la séquence minimale RRE1x (voir figure 5) :
Les oligonucléotides (sens 5'→3') RRE1xa (TCGACTATGGGCGCAGCGTCAATGACGCTGACGGTACAGG CC) et RRE1xb (TCGAGGCCTGTACCGTCAGCGTCATTGACGCTGCGCCCAT AG) purifiés, sont phosphorylés puis hybridés entre eux, conformément aux protocoles ci-après.

* synthèse et purification des oligonucléotides :
Les oligonucléotides sont synthétisés sur un synthétiseur Applied Biosystem, selon la méthode à la phosphoramidite. Non détritylés après synthèse, ils sont lyophilisés puis purifiés sur colonnes à usage unique NENsorb prep® (DuPont Co.), selon le protocole du fabricant. Les oligonucléotides tritylés sont ainsi retenus de façon sélective sur la colonne, puis ils sont élués après une hydrolyse acide (0,5 % (p/v) TFA) de la fonction trityle. Après une nouvelle lyophilisation, ils sont repris par du tampon TE (Tris-HCl 10 mM, pH 8, et EDTA 1 mM). Les concentrations des solutions obtenues sont déterminées par la mesure de l'absorbance à 260 nm sachant qu'une absorbance d'une unité correspond à 37 μg d'ADN.
* Phosphorylation :
Le milieu réactionnel, d'un volume final de 20 μl, contient 2 μg d'oligonucléotide 70 mM de tampon Tris-HCl pH 7,6, 0,75 mM d'ATP, 10 mM de chlorure de magnésium, 5 mM de DTT et 5 unités de phosphonucléotide kinase du phage T4 (PNK).
Après une incubation d'une heure à 37°C, l'enzyme est inactivée par une incubation à 65°C pendant 10 minutes.
Lors des hybridations radioactives, le groupement ajouté contient du $^{32}$P. Dans ce cas, 30 pmoles d'oligonucléotide sont phosphorylées (sachant que 0,1 μg = 250/L en pmoles avec L = longueur de l'oligonucléotide) dans 10 μl de tampon 25 mM Tris-HCl pH 7,6, 6 mM MgCl$_2$, 2,5 mM DTT contenant 2 μl de $^{32}$P-γ-ATP (activité spécifique>5 000 Ci/mmol) et 4 U de polynucléotide kinase. Après dénaturation thermique de l'enzyme, l'excès d'ATP radioactif est éliminé par précipitation à l'éthanol. L'efficacité d'incorporation du $^{32}$P est ensuite vérifiée par comptage à l'aide d'un compteur à scintillation (Packard Liquid Scintillation Analyzer).
* Hybridation des oligonucléotides entre eux :
2 μl de chaque oligonucléotide phosphorylé sont incubés pendant 10 min à 80°C dans un volume final de 20 μl de NaCl 100 mM. L'hybridation est ensuite obtenue par un refroidissement lent jusqu'à température ambiante.
* Préparation d'ADN plasmidiques et techniques de clonage :
Les techniques générales d'analyse et de clonage d'ADN sont celles décrites par MANIATIS et al. (1982). L'extraction de l'ADN plasmidique a été effectuée selon la méthode de lyse alcaline. Les enzymes de restriction (Pharmacia, BRL, Appligene, Eurogenthec) sont utilisées selon les recommandations des fournisseurs.
Pour les clonages, les fragments d'ADN obtenus après digestions enzymatiques sont séparés sur gel d'agarose « low melting » (Seaplaque®, Nusieve) TAE, puis extraits sur une résine (Kit Magic® prep). Les vecteurs linéarisés ayant la possibilité de se refermer sur eux-mêmes sont déphosphorylés par traitement à la phosphatase alcaline. La phosphatase utilisée est la phosphatase alcaline d'*E. coli* à 141 U/ml commercialisée par Pharmacia.
Cette enzyme se conserve dans de l'eau ou dans son tampon de réaction (Tris-HCl 50 mM pH 8, ZnCl$_2$ 1 mM).
L'ADN à déphosphoryler est remis en suspension dans 50 μl de tampon de réaction et incubé 30 min à 60°C. La réaction est stoppée par ajout de 0,1 % SDS et de 100 μg/ml de protéinase K pendant 50 min à 37°C. L'ADN est alors extrait au phénol, précipité et repris dans 20 μl d'eau stérile. Le clonage est réalisé en mélangeant 4 μl de vecteur déphosphorylé, 10 μl du fragment RRE1x double brin, en présence d'une unité Weiss de ligase T4 et de son tampon (DTT 10 mM, Tris-HCl 50 mM pH 7,6, MgCl$_2$ 10 mM, ATP 1 mM).
L'incubation du mélange de ligation est effectuée pendant une nuit à 16°C. La transformation des cellules compétentes est réalisée par électroperméabilisation selon Dower WJ. et al. (NAR, 1988, **16**, 6127-6145).
La formation du RRE1x double-brin fait apparaître un site de restriction Sall (site de restriction b selon l'invention), à l'extrémité 5' et un site Xhol (site de restriction a selon l'invention), à l'extrémité 3'. Ce mélange d'hybridation est incubé avec le plasmide BSKS, linéarisé par ces mêmes enzymes, en présence de ligase.
* Détection des clones comportant l'insert RRE1x : hybridation radioactive sur colonies :
Après transformation dans TG1, les différents clones sont analysés, pour sélectionner ceux qui possèdent effectivement l'insert correspondant à RRE1x, de 42 pb.

Ce clonage est vérifié par hybridation *in situ,* effectuée sur les différents clones obtenus, à l'aide d'une sonde radioactive formée de l'oligonucléotide RRE1xb marqué en γ au $^{32}$P.

Lors du clonage du fragment synthétique RRE1x de 42 pb dans BSKS, le criblage est réalisé par hybridation *in situ* selon la méthode de Grunstein et Hogness (1975).

Après transformation de la souche TG1 par le produit de ligation obtenu ci-dessus et étalement sur milieu sélectif, une centaine de colonies obtenues sont repiquées sur boîte et mises en culture une nuit à 37°C. Une membrane de nylon (Pall Biodyne® A, diamètre=85 mm) est déposée sur la boîte.

Après 3 à 5 min d'adsorption, la membrane est enlevée et déposée sur du papier Whatman®, afin de sécher, 30 min à température ambiante. La membrane est alors déposée à la surface d'une solution dénaturante de NaOH 0,5 M (1 à 3 minutes), puis immergée successivement dans une solution de Tris 1 M pH 7,5, NaCl 1,5 M et Tris 0,5 M pH 7,5, NaCl 1,5 M. Elle est ensuite délicatement séchée entre deux feuilles de papier absorbant, puis l'ADN est fixé deux heures sous vide à 80°C ou 10 secondes au Cross-linker.

La sonde est obtenue par le marquage de l'oligonucléotide RRE1xa. L'oligonucléotide est radio-marqué par phosphorylation (par la T4 Polynucléotide Kinase et du 32P-γ-ATP). L'hybridation est effectuée par ajout de la sonde, à raison de $10^6$ cpm/ml de mélange d'hybridation toute une nuit à une température définie. Cette température est calculée selon les relations empiriques établies par LATHE R. (J. Mol. Biol., 1985, **183**, 1-12), soit :

$$\theta2XSSC(°C) = 94-820/L-1.2.(100-H)$$

avec

H =   100(L-M)/L
L :   longueur de l'oligonucléotide,
H :   homologie de séquences entre la sonde et l'ADN,
M :   nombre de bases non appariées.

Le filtre est ensuite lavé 5 minutes avec une solution contenant du SSC2X, SDS 0,1 %, sous agitation à température ambiante, afin d'éliminer la sonde non parfaitement appariée (le SSC20X est une solution contenant 173,5 g/l de NaCl, 8,2 g/l de citrate de sodium ajustée à pH = 7 avec une solution de soude 10 N).

La révélation se fait par autoradiographie, pendant une nuit à -80°C (Film Kodak).

*   Résultats :

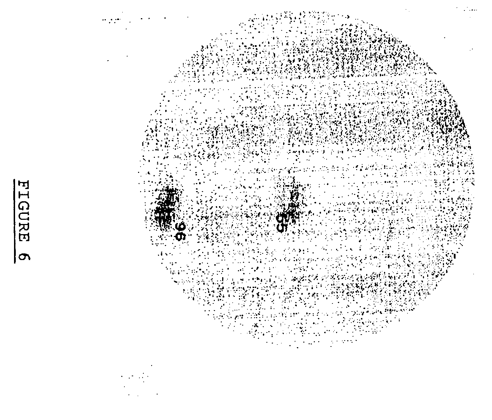

Sur 100 clones ainsi analysés, deux répondent à la sonde et contiennent donc l'insert de 42 pb. Il s'agit des clones 55 et 96 (voir figure 6), représentés sur le film autoradiographique. Cette présence de l'insert a été vérifiée par la perte du site NdeI, situé entre les deux sites de clonage. Les sites de restriction SalI et XhoI étant compatibles, on peut penser que les 98 clones négatifs correspondent à la forme recircularisée du plasmide BSKS ouvert par ces enzymes.

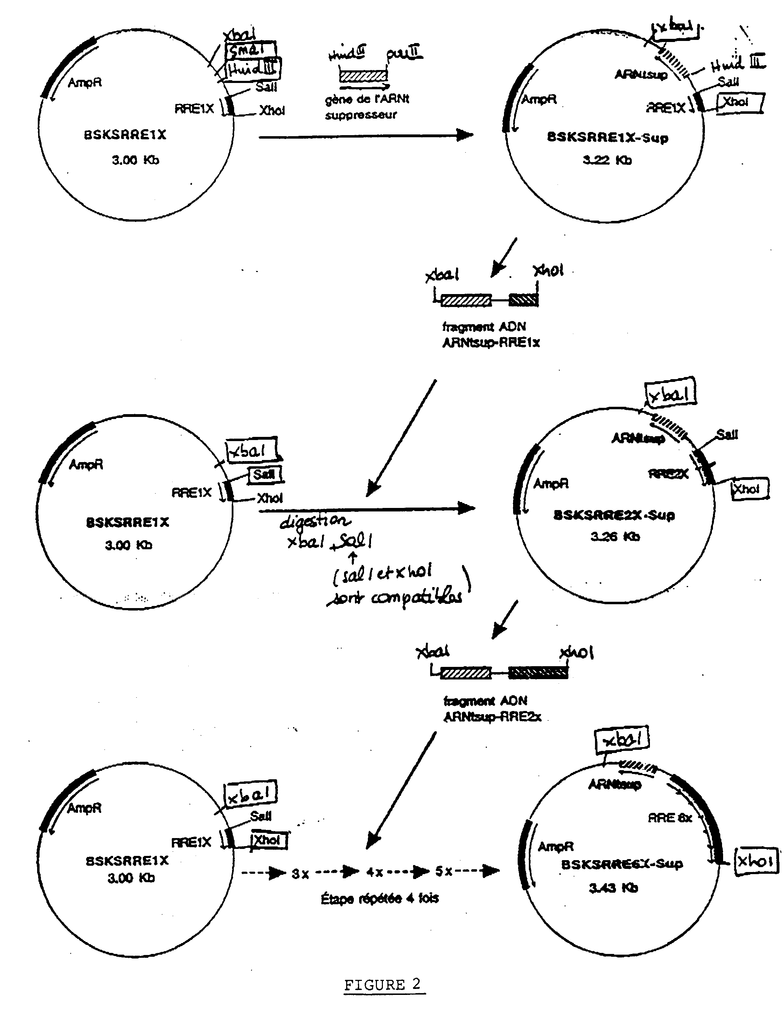

Seule l'orientation permettant de recréer les sites de restriction est acceptable. Après digestion des deux clones positifs par les enzymes ayant servi au clonage, SalI et XhoI, seul le clone 96 possède la bonne orientation. Ce clone appelé BSKS-RRE1x, illustré à la figure 2, est le point de départ pour la polymérisation de RRE.

- Polymérisation de RRE1x

Pour faciliter l'obtention des différents polymères, le vecteur BSKS-RRE1x a été modifié par l'ajout d'un fragment d'ADN, qui porte une nouvelle sélection.

Ce fragment d'ADN est le gène d'un ARNt$^{Phé}$ suppresseur d'*E. coli,* qui est indispensable à la suppression de la mutation ambre portée par une souche d'*E. coli* XAC-1.

Cette mutation affecte un gène, ArgE, codant pour une enzyme du métabolisme de l'arginine. Sans la suppression de cette mutation et sans ajout d'arginine dans le milieu de culture, la souche XAC-1 (mut⁻) ne peut survivre. En effet, cet ARNt suppresseur reconnaît le codon ambre du gène ArgE, en apportant un résidu phénylalanine à la place, dans la séquence protéique, restaurant alors la forme sauvage de l'enzyme.

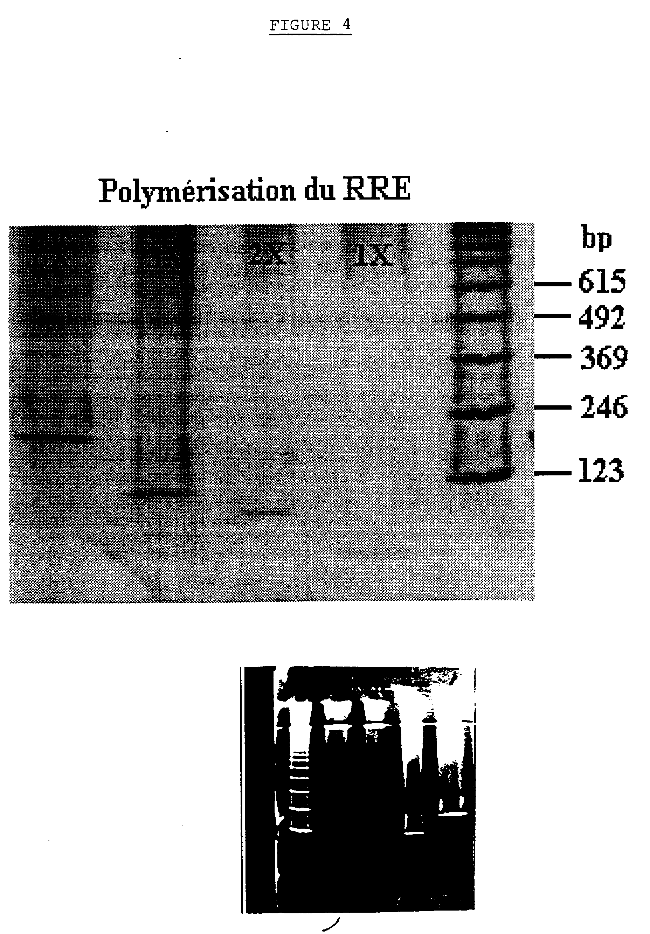

Le gène de l'ARNt$^{Phé}$ suppresseur d'*E. coli* est obtenu à partir du vecteur pGFIB-1, par action des enzymes PvuII et HindIII. Ce fragment de 270 pb est inséré dans le vecteur BSKS-RRE1x, en amont du RRE1x, grâce aux sites de restriction SmaI et HindIII. Les sites PvuII et SmaI n'étant pas compatibles, l'insertion est vérifiée par coupure par XbaI (site <u>d</u>) et HindIII (site <u>c</u>), générant un fragment approximatif de 280 pb. Le fragment XbaI-XhoI, contenant le RRE1x et le suppresseur (fragment 1x+s) est introduit dans le vecteur BSKS-RRE1x non modifié (clone 96), ouvert par les enzymes de restriction XbaI (site <u>d</u>) et SalI (site <u>b</u>). Ce clonage permet l'insertion d'une nouvelle copie de RRE1x, adjacente à la première copie de RRE1x (voir figure 2), pour former le vecteur BSKS-RRE2x+s. Cette méthode de construction est répétée sur le plasmide BSKS-RRE2x, pour former le BSKS-RRE3x, puis ainsi de suite, jusqu'à réaliser le vecteur BSKS-RRE6x (voir figure 2). La présence du polymère 6x est vérifiée par séquençage et par électrophorèse (figure 4), à partir du BSKS-RRE6x.

La présence du suppresseur d'*E. coli* comme pression de sélection permet d'obtenir, à chaque étape de clonage, 100 % de clones ayant la construction désirée, après transformation de la souche d'*E. coli* XAC-1 (argE, lacZ$_{am}$) obtenue. La suppression de la mutation du codon ambre présente sur le gène LacZ entraîne la production d'une β-galactosidase active, qui permet l'apparition de colonies bleues sur un milieu contenant l'indicateur X-gal (5-bromo-4-chloro-3-indolyl-β, D-galactopyranoside) ; la suppression de la mutation du codon ambre sur le gène ArgE entraîne l'apparition du phénotype Arg+.

La figure 2 illustre les différents étapes de la polymérisation et les différents polymères obtenus (1x, 2x, 3x et 6x).=

**EXEMPLE 2 : Mise en oeuvre du procédé pour préparer une séquence polymérique, à base de la séquence d'ARN cible de Rev, le RRE : obtention de la séquence polymérique RRE6x dans une souche d'E.coli XAC-1recA.**

On procède comme à l'exemple 1 ; toutefois le vecteur RRE6x+s sert à transformer une souche d'E. coli XAC-1 *recA.*

L'introduction de la mutation recA dans les souches XAC-1 d'E. coli permet d'éviter que des événements de recombinaison ne viennent perturber la sélection, conformément au protocole suivant :

\* Transduction :

Afin d'éviter tout phénomène de recombinaison au cours de la polymérisation du RRE, la souche XAC-1 a été modifiée et rendue *recA*- par transduction du phage P1-*recA*-.

Les gènes bactériens peuvent non seulement être transférés par conjugaison ou par transposition, mais ils peuvent également être transportés passivement d'une bactérie à l'autre par des particules phagiques, ce processus étant appelé transduction. Cela se produit lorsqu'une particule virale (un phage transducteur) encapsule accidentellement des portions du chromosome de l'hôte. Dans le cas présent, il s'agit de la partie chromosomique portant le fragment délété du gène *recA* associé au gène *Srl* (nécessaire à l'utilisation du sorbitol) contenant un transposon lui conférant la résistance à la tétracycline. Lorsque le phage transducteur s'attache à une cellule hôte, le fragment de chromosome bactérien est injecté dans cette cellule. Ce fragment peut alors subir un *crossing over* avec le chromosome hôte. Lorsque ce phage transducteur (phage P1) est cultivé sur une souche génétiquement différente *(recA+)* de la souche postérieurement infectée (*recA*-) par le phage, il peut s'en suivre la formation d'une bactérie génétiquement transformée (*recA*-). Le segment du chromosome bactérien porté par une seule particule transductrice est relativement court, de sorte que seul les gènes très proches les uns des autres seront inclus dans la même particule transductrice. C'est pourquoi cette souche, *recA*-, nouvellement formée est sélectionnée sur milieu sélectif additionné de tétracycline. Ce phénotype *recA*- peut être vérifié par la sensibilité importante de la souche aux U.V.

\* Préparation de phages P1-*recA*- :

La souche donneuse *recA*- est mise en culture dans 5 ml de LB toute une nuit à 37°C. La culture est diluée, de façon à obtenir une D.O.$_{578}$ comprise entre 0,05 et 0,1, dans 5 ml de LB, additionné de 0,2 % de glucose et de 25 μl de CaCl$_2$ 1 M, puis répartie en trois fioles et mise sous agitation 30 minutes à 37°C. 75 μl de stock de phage P1 sont rajoutés dans la première fiole, 150 μl dans la seconde alors que la troisième sert de témoin. La culture infectée est remise à 37°C jusqu'à lyse complète (2 à 3 heures). L'ADN de phage est purifié par ajout de 100 μl de chloroforme dans les fioles, le mélange est agité fortement et centrifugé 10 minutes à 8 000 rpm à 4°C. Le surnageant constitue le stock de phage P1-*RecA*- pour la transduction.

\* Infection d'une souche *recA+* par le phage P1-*recA*- :

La souche receveuse XAC-1 *(recA+)* est cultivée dans 5 ml de LB toute une nuit à 37°C. Après centrifugation 10 minutes à 8 000 rpm, le culot est repris dans 10 ml de tampon MC (MgSO$_4$ 0,1 M ; CaCl$_2$ 5 mM) et incubé 10 min à 37°C sous agitation. 100 μl de cellules ainsi préparées sont mélangées avec 100, 10 ou 1 μl de stock de phage P1-*recA*- confectionné plus haut, un tube contenant uniquement les cellules sert de témoin. 20 minutes à l'étuve suffisent à l'absorption des phages. 200 μl de citrate de sodium 1 M sont ajoutés dans les tubes puis 2,5 ml de Top Agar. Le mélange est rapidement coulé sur les boîtes contenant la sélection portée par le phage (tétracycline). Les boîtes sont laissées 48 heures à l'étuve à 37°C.

**EXEMPLE 3 : Construction de séquences régulatrices pour le contrôle de l'expression de gènes eucaryotes.**

Le procédé selon l'exemple 1 ou l'exemple 2 est mis en oeuvre pour construire une séquence comprenant, en nombre répété, une série de séquences courtes (séquences régulatrices) en remplaçant la séquence RRE par l'une des séquences citées ci-après ; en effet, un grand nombre de gènes eucaryotes peuvent être contrôlés par l'adjonction en amont du gène d'une série de séquences courtes, répétées en tandem, le nombre de répétitions déterminant le taux de contrôle du gène. C'est par exemple le cas des séquences reconnues par le facteur de régulation GAL4 dans la levure, qui font partie de ce que l'on nomme généralement les *Upstream Activating Sequences* (UAS). Dans les cellules de mammifères, on trouve de très nombreuses séquences régulatrices qui sont répétées (souvent 3 ou 4 fois) en amont de la séquence promotrice. Par exemple, la séquence (GGGACTTTCCGC) fixant le facteur NF-KB, les séquences fixant les facteurs AP1, Sp1, COUP, USF, etc...

Le procédé selon l'invention permet d'obtenir de telles séquences répétées de manière simple.

**EXEMPLE 4 : Gènes de protéines répétitives : exemple de la gélatine.**

Le procédé selon l'exemple 1 ou l'exemple 2 est mis en oeuvre pour construire une séquence comprenant, en nombre répété n fois, un gène combinant plusieurs séquences codant pour des tripeptides, en remplaçant la séquence RRE par l'une des séquences citées ci-après.

Il y a deux cas de figure :

- le premier concerne les gènes codant pour des protéines telles que la soie, la kératine ou le collagène et la gélatine. Il s'agit de courtes séquences codant pour 3 à 6 acides aminés et qui sont hautement répétées ou semi-répétées.

   Par exemple, la gélatine est constituée de chaînes d'acides aminés du type (Gly-X-Pro) n fois qui lui confèrent ses propriétés plastiques et physico-chimiques particulières. La synthèse d'un gène combinant plusieurs séquences codant pour des tripeptides différents (Gly-Ala-Pro), (Gly-Asp-Pro), (Gly-Val-Pro), etc... répétées n fois peut être obtenue par le procédé selon l'invention. La protéine ainsi obtenue constitue alors une gélatine aux propriétés originales qui trouve des applications spécifiques en photographie aussi bien qu'en chirurgie.

- autre cas de figure : certaines protéines comportent des courts segments répétés qui ont généralement pour fonction l'adhésion à un tissu ou à une structure particulière. On peut utiliser des fragments de gènes obtenus par le procédé selon l'invention pour inclure de telles séquences dans des protéines données. Ceci inclut l'addition d'« étiquettes » en N ou C-terminal (répétition d'un peptide antigénique de 6 à 10 résidus) servant à fixer un anticorps monoclonal, pour la purification par immuno-affinité ou pour le repérage dans les tissus.

   Ainsi que cela ressort de ce qui précède, l'invention ne se limite nullement à ceux de ses modes de mise en oeuvre, de réalisation et d'application qui viennent d'être décrits de façon plus explicite ; elle en embrasse au contraire toutes les variantes qui peuvent venir à l'esprit du technicien en la matière, sans s'écarter du cadre, ni de la portée, de la présente invention.

## EP 0 771 872 A1

**Revendications**

1. Procédé de polymérisation d'une séquence nucléique par une méthode de clonage, lequel procédé est caractérisé en ce qu'il comprend essentiellement les étapes suivantes :

9

(1) coupure d'un vecteur de clonage circulaire comprenant deux sites de restriction, a et b, donnant des extrémités compatibles entre elles et deux sites de restriction $\underline{c}$ et $\underline{d}$, proches des sites de restriction $\underline{a}$ et $\underline{b}$, à l'aide d'au moins une enzyme de restriction, apte à effectuer ladite coupure au niveau des sites $\underline{a}$ et $\underline{b}$,

(2) introduction par ligation de la séquence nucléique à polymériser dans le vecteur linéarisé obtenu en (1), entre les deux sites de restriction, $\underline{a}$ et $\underline{b}$ (obtention du vecteur 1x, c'est-à-dire contenant une seule copie de la séquence à polymériser),

(3) coupure du vecteur recircularisé obtenu en (2), à l'aide d'au moins une enzyme de restriction, apte à effectuer ladite coupure au niveau des sites de restriction $\underline{c}$ et $\underline{d}$

(4) introduction par ligation, dans le vecteur linéarisé obtenu en (3), entre les deux sites de restriction $\underline{c}$ et $\underline{d}$, d'un gène codant pour un ARNt suppresseur (obtention du vecteur 1x+s, contenant une seule copie de la séquence à polymériser et le gène codant pour un ARNt suppresseur) et sélection des clones portant l'insert 1x+s selon la bonne orientation, par transformation de bactéries convenables,

(5) coupure du vecteur obtenu en (4) à l'aide d'au moins une enzyme de restriction, apte à effectuer ladite coupure au niveau des sites $\underline{a}$ et $\underline{d}$,

(6) introduction par ligation du fragment $\underline{a}$-$\underline{d}$ obtenu en (5) (fragment 1x+s) entre les sites $\underline{b}$ et $\underline{d}$ d'un vecteur 1x tel qu'obtenu en (2), après coupure de ce vecteur 1x à l'aide d'au moins une enzyme de restriction, apte à effectuer ladite coupure au niveau desdits sites $\underline{b}$ et $\underline{d}$ (obtention du vecteur 2x+s) et sélection des clones portant l'insert 2x+s dans la bonne orientation, par transformation de bactéries convenables,

(7) introduction par ligation du fragment obtenu en (6) (fragment 2x+s) entre les sites b et d d'un vecteur 1x tel qu'obtenu en (2), après coupure de ce vecteur 1x telle que définie en (6)(obtention du vecteur 3x+s),

(8) répétition de l'étape (7) par insertion du fragment nx+s obtenu en dernier lieu dans un vecteur 1x, jusqu'à obtention d'une séquence comportant n+1 fragments identiques à la séquence nucléique à polymériser,

(9) transformation d'une souche bactérienne avec le vecteur obtenu en (8), laquelle souche bactérienne comporte une mutation mut$^-$, de telle sorte que son développement ne soit possible qu'en présence dudit ARNt suppresseur (c'est-à-dire en présence du vecteur nx+s) et sélection des souches présentant le caractère mut$^+$ et

(10) extraction du polymère (n+1)x, à partir desdites souches sélectionnées.

**2.** Procédé selon la revendication 1, caractérisé en ce que les sites de restriction sont de préférence sélectionnés

- pour ce qui concerne les sites de restriction a et b, de préférence différents et aux extrémités compatibles, parmi tous les sites correspondant à des enzymes coupant l'ADN à bouts francs, tels que Pvu II, Eco RV, Hinc II, Sma I, Sna BI ou Ssp I, les sites de coupure des enzymes donnant des extrémités cohésives compatibles, mais dont la fusion ne régénère pas un site qui peut être recoupé par l'une des deux enzymes, tels que Eco RI et Mun I ; Apa LI et Sfc I ; Bam HI et Bcl I, Bgl II ou Bst YI ; Sal I et Xho I ; Nco I et Bsp HI ou Afl III ; Xma I et Age I ; Ngo MI et Bsp EI ; Xba I et Nhe I, Avr II ou Spe I ; Bss HII et Mlu I ou Dsa I ; Bst BI et Cla I, Acc I, Nar I, ou Psp 1406 I ; Pst I et Nsi I ;
- pour ce qui concerne les sites de restriction $\underline{c}$ et $\underline{d}$, de préférence différents des sites $\underline{a}$ et $\underline{b}$, parmi n'importe quel site de restriction, à condition qu'il ne soit pas inclus dans la séquence de l'ARNt suppresseur.

**3.** Procédé selon la revendication 2, caractérisé en ce que le site $\underline{b}$ et le site $\underline{c}$ sont confondus.

**4.** Procédé selon la revendication 2, caractérisé en ce que le site $\underline{a}$ est de préférence un site de restriction XhoI, le site $\underline{b}$ est de préférence un site de restriction SalI, le site $\underline{c}$ est de préférence un site de restriction HindIII et le site $\underline{d}$ est de préférence un site de restriction XbaI.

**5.** Procédé selon l'une quelconque des revendications 1 à 4, caractérisé en ce que ledit ARNt suppresseur est sélectionné parmi les ARNt suppresseurs d'ocre, d'opale, frameshift ou d'ambre.

**6.** Procédé selon l'une quelconque des revendications 1 à 5, caractérisé en ce que ladite souche bactérienne est sélectionnée parmi les souches d'*Escherichia coli* portant une mutation d'ambre à la fois dans un gène essentiel et dans un gène rapporteur et les souches d'*Escherichia coli* portant une mutation d'ambre à la fois dans un gène essentiel et dans un gène rapporteur et une mutation recA.

**7.** Procédé de construction d'échelles régulières de molécules d'ADN, caractérisé en ce qu'il comprend :

- la préparation de n vecteurs selon le procédé selon l'une quelconque des revendications 1 à 6, lesdits n vecteurs portant chacun un nombre défini de copies de la séquence initiale (de 1 à n) et

- la coupure desdits différents vecteurs par les deux enzymes compatibles $\underline{a}$ et $\underline{b}$, qui encadrent la séquence polymérisée.

8. Procédé d'inclusion de séquences répétitives d'ADN dans des vecteurs ou des génomes, caractérisé en ce qu'il comprend la préparation d'une séquence d'acide nucléique polymérisée à l'aide du procédé selon l'une quelconque des revendications 1 à 6, la séparation du polymère et son inclusion dans le vecteur convenable.

FIGURE 1

FIGURE 2

FIGURE 3

FIGURE 4

# Polymérisation du RRE

XhoI

5' TCGACTATGGGCGCAGCGTCAATGACGCTGACGGTACAGGCC    3'
      GATACCCGCGTCGCAGTTACTGCGACTGCCATGTCCGGAGCT
SalI

FIGURE 5

FIGURE 6

Office européen
des brevets

## RAPPORT DE RECHERCHE EUROPEENNE

Numero de la demande

EP 96 40 1959

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| Y | US-A-4 403 036 (HARTLEY JAMES L ET AL) 6 Septembre 1983 <br> * exemple 1 * <br> --- | 1-8 | C12N15/10 <br> C12N15/66 <br> C12N15/70 |
| Y | SAMBROOK J. ET AL.: "Molecular cloning, a laboratory manual" <br> 1989 , COLD SPRING HARBOR LABORATORY PRESS <br> XP002002887 <br> * page 1.6, alinéa 5 * <br> * page 1.19 * <br> --- | 1-8 | |
| Y | WO-A-90 05177 (SYNTRO CORP) 17 Mai 1990 <br> * page 18, ligne 32 - ligne 33 * <br> --- | 1-8 | |
| A | WO-A-88 05082 (ALLIED CORP) 14 Juillet 1988 <br> * revendication 1 * <br> ----- | 1-8 | |

| | | | DOMAINES TECHNIQUES RECHERCHES (Int.Cl.6) |
|---|---|---|---|
| | | | C12N |

Le présent rapport a été établi pour toutes les revendications

| Lieu de la recherche | Date d'achèvement de la recherche | Examinateur |
|---|---|---|
| LA HAYE | 6 Janvier 1997 | Gurdjian, D |

### LISTE DE SEQUENCES

(1) INFORMATIONS GENERALES:

    (i) DEPOSANT:
        (A) NOM: CENTRE NATIONAL DE LA RECHERCHE SCIENTIFIQUE-CNRS
        (B) RUE: 3 RUE MICHEL ANGE
        (C) VILLE: PARIS
        (E) PAYS: FRANCE
        (F) CODE POSTAL: 75794 CEDEX 16

    (ii) TITRE DE L' INVENTION: PROCEDE DE POLYMERISATION DE SEQUENCES D'ACIDES NUCLEIQUES ET SES APPLICATIONS.

    (iii) NOMBRE DE SEQUENCES: 2

    (iv) FORME DECHIFFRABLE PAR ORDINATEUR:
        (A) TYPE DE SUPPORT: Floppy disk
        (B) ORDINATEUR: IBM PC compatible
        (C) SYSTEME D' EXPLOITATION: PC-DOS/MS-DOS
        (D) LOGICIEL: PatentIn Release #1.0, Version #1.30 (OEB)

    (vi) DONNEES DE LA DEMANDE ANTERIEURE:
        (A) NUMERO DE LA DEMANDE: FR 95 10872
        (B) DATE DE DEPOT: 15-SEP-1995

(2) INFORMATIONS POUR LA SEQ ID NO: 1:

    (i) CARACTERISTIQUES DE LA SEQUENCE:
        (A) LONGUEUR: 42 paires de bases
        (B) TYPE: nucléotide
        (C) NOMBRE DE BRINS: simple
        (D) CONFIGURATION: linéaire

    (ii) TYPE DE MOLECULE: Autre acide nucléique
        (A) DESCRIPTION:  /desc = "OLIGONUCLEOTIDE"

    (xi) DESCRIPTION DE LA SEQUENCE: SEQ ID NO: 1:

TCGACTATGG GCGCAGCGTC AATGACGCTG ACGGTACAGG CC        42

(2) INFORMATIONS POUR LA SEQ ID NO: 2:

    (i) CARACTERISTIQUES DE LA SEQUENCE:
        (A) LONGUEUR: 42 paires de bases
        (B) TYPE: nucléotide
        (C) NOMBRE DE BRINS: simple
        (D) CONFIGURATION: linéaire

    (ii) TYPE DE MOLECULE: Autre acide nucléique
        (A) DESCRIPTION:  /desc = "OLIGONUCLEOTIDE"

    (xi) DESCRIPTION DE LA SEQUENCE: SEQ ID NO: 2:

TCGAGGCCTG TACCGTCAGC GTCATTGACG CTGCGCCCAT AG        42